# EUROPEAN PATENT APPLICATION

(11) **EP 1 053 716 A1**
(43) Date of publication of application: **22.11.2000**
(21) Application number: 99660078.9
(22) Date of filing: 17.05.1999
(51) Int. Cl.: A61B 7/04

(54) **Electronic stethoscope**

(71) Applicant: OOO "Tsvetochnoye", Leningradskaya Oblast 188900 (RU)
(72) Inventor: Potapov, Anatoly Ivanovich, St. Petersburg 191126 (RU); Polyakov, Vitaly Yevgenyevich, St. Petersburg 194356 (RU)
(74) Representative: Papula, Antti

(57) **Abstract**

An electronic stethoscope for examination of acoustically active objects comprises an acoustical-electrical transducer (1) capable of being brought into an acoustic contact with the object surface. The transducer (1) is provided with a piezoelectric acceleration sensor (2) adapted for sensing accelerations transferred to it from the object and arising under the action of acoustic oscillations generated in said object. The sensor (2) is provided with at least one piezoelectric element (3) made of porous ceramic material and loaded with an inertial mass (4).

## Description

### Field of the Invention

The invention relates to acoustic diagnostic means and more specifically, to electronic stethoscopes intended for examination of acoustically active objects.

### Background of the Invention

Stethoscopes enjoy a large use in medicine for listening to, or auscultation of, heart tones, cardiac and respiratory murmurs and other sounds taking place within a human organism, as well as in engineering, e.g. with the purpose of diagnosing motors and other machines. Conventional stethoscopes, which are not provided with sound amplification means, suffer from a drawback consisting in their low sensitivity.

In order to increase the sensitivity, electronic stethoscopes have been suggested, which stethoscopes comprise a microphone for converting acoustic vibrations into an electric signal to be supplied to the input of an amplifier whose output is connected to a telephone for reconverting the amplified signal into sound. When auscultating an object, the microphone of the electronic stethoscope is brought into contact with the object surface. In such stethoscopes, the acoustical signal is usually fed from the telephone through sound-conducting tubes to a pair of olives to be inserted into the user's ears.

An electronic stethoscope of this type, which can be used for auscultation of acoustically active objects and comprises a transducer capable of being brought into an acoustic contact with the object surface for converting acoustic vibrations into an electric signal, is, e.g., described in the advertisement leaflet "New Electronic Stethoscope. LAB 3001" issued by the Labtron Scientific Corp. In this known stethoscope, the function of an acoustical-electrical transducer is performed by a microphone.

A disadvantage of this known electronic stethoscope consists in its poor immunity to outside sounds coming to it from various directions through the air and impeding the auscultation of the object of interest. This disadvantage is due to the fact that a microphone, being an acoustic pressure sensor, is highly sensitive both to the heard object surface oscillations whose sources are located inside the object, and to spurious sound oscillations received through the air from extraneous sources. Such a low noise immunity of the known stethoscope can make difficult its use in real service conditions, e.g. when auscultating a patient in a common ward of a hospital against the background of ambient noises. Furthermore, the noise immunity of the known stethoscope is hindered by the broad beam pattern of the microphone because it makes difficult separate study of signals coming from different acoustic sources located inside the object under examination, such as human internals.

### Summary of the Invention

Accordingly, it is an object of the invention to enhance the immunity of an electronic stethoscope to extraneous acoustic oscillations.

With this object in view, there is proposed an electronic stethoscope for examination of acoustically active objects, comprising an acoustical-electrical transducer capable of being brought into an acoustic contact with the object surface, wherein, according to the invention, said transducer includes a piezoelectric acceleration sensor adapted for sensing accelerations transferred to it by the object and arising under the action of acoustic oscillations generated in said object.

Piezoelectric acceleration sensors are largely used in various equipment as sensing members of accelerometers. Such a sensor has a high sensitivity to the vibrations of the object surface with which it is brought into contact. At the same time, it is well known that piezoelectric acceleration sensors are in fact insensitive to acoustic oscillations coming through air because, unlike standard microphones, they are not fitted with a diaphragm or any other member responding to variations in the air pressure. Therefore, owing to the use of a piezoelectric acceleration sensor, it becomes possible to improve the immunity of an electronic stethoscope to extraneous acoustic oscillations. In addition, piezoelectric acceleration sensors exhibit a relatively high directivity, thus further enhancing the noise immunity and allowing separate study of acoustic signals coming from different sound sources located inside the object under examination.

The piezoelectric acceleration sensor preferably includes at least one piezoelectric element loaded with an inertial mass.

According to a preferred embodiment of the invention, said at least one piezoelectric element is made of porous ceramic material that is unsuitable for conventional acceleration sensors used in accelerometers due to its lower strength as compared with solid ceramics. However, when used in a sensor of an electronic stethoscope where it is subjected to only insignificant accelerations, such a porous ceramic material will generally have a quite acceptable strength, whereas its lower density and porous texture make it possible to substantially increase the sensitivity of the sensor in the audio frequency band, as well as to reduce its equivalent output resistance, thereby facilitating its matching with the amplifier input within a large frequency range and enabling a decrease in the intrinsic noise level of the electronic stethoscope.

The proportion of pores volume in the porous ceramic material forming the piezoelectric element is preferably at least about 20 % of the overall ceramics volume.

According to one of the preferred embodiments of the invention, the piezoelectric acceleration sensor comprises a plurality of piezoelectric elements circumferentially spaced at regular intervals on the inertial mass and electrically connected with each other. With such an arrangement, it is possible to reduce the width of the sensor beam pattern, thus allowing a more selective study of oscillations coming from various points inside the object.

The piezoelectric acceleration sensor may also comprise at least two piezoelectric elements mounted on the inertial mass one over the other and electrically connected with each other, thereby increasing the sensitivity of the stethoscope.

The piezoelectric elements are preferably electrically connected in parallel in order to reduce the sensor output resistance.

The inertial mass may be a cup bearing on its bottom at least one piezoelectric element, so that the centers of gravity of said inertial mass and said piezoelectric element are brought closer to one another whereby the sensor sensitivity is increased.

In accordance with one of the embodiments, the transducer forming part of the electronic stethoscope is provided with a sound-conducting rod one end of which is intended to be brought into contact with the object to be examined and the other end of which is in an acoustic contact with the piezoelectric acceleration sensor.

The transducer surface intended to be brought into an acoustic contact with the surface of the object is preferably equipped with an elastic protective cover.

The electronic stethoscope preferably comprises means for frequency filtering the signal delivered by the sensor, while the filtering means may have a switch-selectable passband.

The electronic stethoscope preferably comprises means for amplifying the signal delivered by the sensor; the amplifying means may have a controllable gain.

The electronic stethoscope preferably further comprises means for converting the signal amplified by the above-mentioned amplifying means into an acoustic signal, and a headset for listening to the acoustic signal generated by said converting means.

Said converting means preferably comprise a telephone, while said headset may be fitted with olives acoustically coupled with said telephone via sound-conducting tubes.

Said converting means may comprise at least one additional telephone connected to a separate headset so as to allow two or more persons to simultaneously hear the acoustic signal.

The electronic stethoscope may further comprise an analog-to-digital converter for converting the signal generated by the sensor into a digital form, while a digital signal analysis unit may be connected to the output of said analog-to-digital converter.

In addition, the electronic stethoscope may be adapted for supplying the signal generated by the acceleration sensor to a computer for the purpose of analysing this signal, and/or comparing it to standard signals, and/or transmitting it via a communication link to another computer.

The electronic stethoscope may also be adapted for feeding the signal generated by the acceleration sensor to an external sound reproducer, and/or sound recorder, and/or graphic recorder.

The electronic stethoscope is preferably provided with output terminals to which the signal generated by the sensor is supplied via an optoisolator.

The piezoelectric acceleration sensor forming part of the electronic stethoscope may be located in a separate unit with the amplifying means being connected to said sensor with the aid of a shielded cable.

### Brief Description of the Drawings

Fig. 1 shows a cross-sectional view of an electronic stethoscope according to the invention.

Fig. 2 is a cross-sectional view of an electronic stethoscope designed in accordance with another embodiment of the invention.

Fig. 3 shows the section along A-A in Fig. 2.

### Detailed Description of the Invention

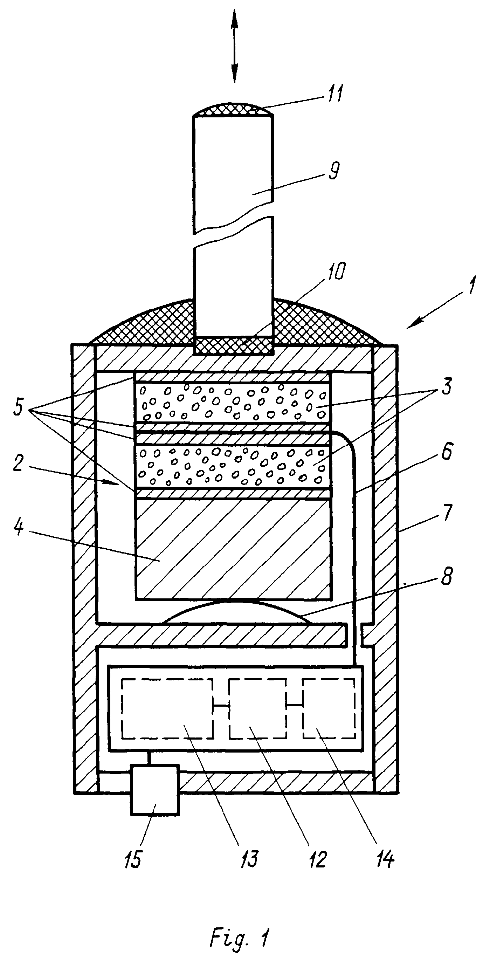

Referring now to Fig. 1, the claimed electronic stethoscope comprises an acoustical-electrical transducer 1 capable of being brought into contact with the surface of an object and provided with a piezoelectric acceleration sensor 2 designed to sense accelerations transferred thereto from the object and arising under the action of sound oscillations generated within the object.

In order to sense accelerations, the piezoelectric sensor 2 shown in Fig. 1 is provided with two piezoelectric elements 3 loaded with an inertial mass 4 which may be made of metal and the weight of which is generally several times greater than that of the piezoelectric elements 3. It is also possible to use a sensor comprising one or several piezoelectric elements.

The piezoelectric elements 3 are formed by cylinders made of polarized porous ceramic material and carrying, on their opposite end faces, electrodes 5.

It would be expedient that the porosity of the ceramic material, i.e. the proportion of pores volume in the total volume of the ceramics, represents at least about 20 %, although the porosity may also be higher, 50 to 60 % for example. The upper limit of the porosity is defined by the requirements imposed for the mechanical strength of the piezoelectric element which decreases with increase in the porosity. As a general rule, the pore size does not exceed hundredths of a millimetre. The material to be used for the piezoelectric element 3 may be the lead zirconate/titanate having, in the absence of any pores, density of about 7·10³ to 7,7·10³ kg/m³. Preferably, the porous lead zirconate/titanate has a density not exceeding 6·10³ kg/m³, more preferably 3,2 to 5,5·10³ kg/m³, the latter density being equivalent to a porosity of 20 to 50 %.

The diameter of the cylindrical piezoelectric elements 3 may, e.g., be about 20 mm and their height, about 10 mm. It shall be evident that piezoelectric elements of some other shape may also be used. The lower limit of the operating frequency range of such a sensor is generally near 10⁻² Hz, while the upper limit, which is determined by the first resonance frequency, in vicinity of 100 kHz.

The piezoelectric elements 3 are mounted one over the other on the inertial mass 4. Their adjoining electrodes are connected to an electric lead 6. The other electrodes of the piezoelectric elements 3 are electrically coupled to a casing 7, one of them being connected to said casing 7 directly and the other, through the inertial mass 4 and a metal spring 8, so that there is provided a parallel connection between said piezoelectric elements 3.

The transducer 1 comprises also a sound-conducting rod 9 one end of which is intended to be brought into contact with the object to be examined (not shown) and the other end of which is connected to the casing 7 via an elastic gasket 10 providing a good acoustic contact with the sensor 2. The sound-conducting rod 9 may be made of plastic material. Its diameter may be about 5 to 10 mm and its length, in the range between 20 cm and 1 to 2 m, depending on the particular application of the stethoscope. The surface of the transducer 1 intended to be brought into an acoustic contact with the object surface is equipped with an elastic protective cover 11 made, e.g., of polyurethane.

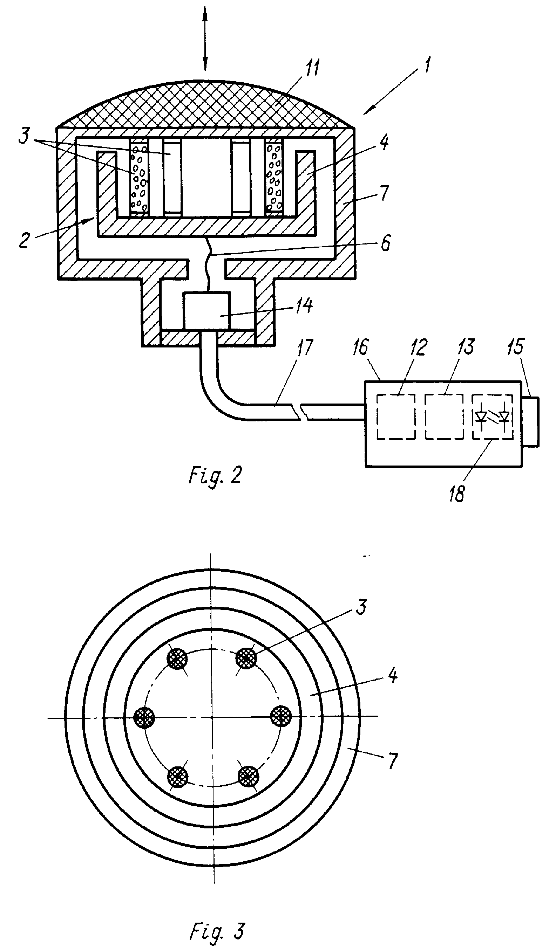

In the embodiment of the invention illustrated in Figs. 2 and 3, the piezoelectric acceleration sensor 2 includes a plurality of piezoelectric elements 3 (six in this particular case) circumferentially spaced at regular intervals on the inertial mass 4. In this case the piezoelectric elements 3 may be formed by cylinders made of a porous ceramic material and having a diameter e.g. of about 5 mm and a height of about 10 mm. As in Fig. 1, the piezoelectric elements 3 are electrically connected with each other in parallel, one of the electrodes of each piezoelectric element being connected to the casing 7, while its other electrode is coupled to the inertial mass 4 connected, in turn, to the electric lead 6. The connection between the electrodes of the piezoelectric elements 3, on the one hand, and the inertial mass 4 and the casing 7, one the other hand, may be effected by soldering or using a current-conducting adhesive. The inertial mass 4 shown in Fig. 2 is made in the form of a cup bearing on its bottom the piezoelectric elements 3. The center of gravity of this inertial mass 4 is preferably coincident with that of the piezoelectric elements 3. In such an arrangement, the weight of the cup 4 may preferably be not greater than the total weight of the piezoelectric elements 3.

The structure of Fig. 2 does not comprise the sound-conducting rod shown in Fig. 1, therefore the elastic protective cover 11 is placed directly onto the surface of the casing 7 intended to be brought into an acoustic contact with the surface of the object of interest.

As schematically shown in Fig. 1, the electric lead 6 serves to connect the sensor 2 with amplifying and filtering means represented by an amplifier 12 with a controllable gain and a frequency filter 13 with a switch-selectable passband, both located in a separate compartment of the casing 7. The amplification control and the passband switching are carried out with the aid of appropriate controls (not shown) which may be positioned on the outer face of the casing 7 or at some distance therefrom. Provided at the input of the amplifier 12 is a matching stage 14, such as a source follower, to match the input resistance of the amplifier 12 with the high output resistance of the piezoelectric elements 3. The output of the filter 13 is coupled to a connector 15.

As shown in Fig. 2, illustrating an alternative embodiment of the electronic stethoscope, the sensor 2, together with the matching stage 14, may be located in a separate transducer unit 1, while the amplifying means 12, placed together with the filter 13 into a separate unit 16, may be connected to the sensor 2 by a shielded cable 17.

Coupled to the connector 15 (Figs. 1 and 2) may be means (not shown) for converting the electrical signal to a sound signal that may, in turn, be coupled to a headset to make possible listening to this sound signal. Such converting means preferably comprise a telephone, while said headset can be provided with olives acoustically coupled to the telephone via sound-conducting tubes, in the same manner as in conventional electronic stethoscopes. Said converting means may include at least one additional telephone to be connected to a separate headset in order to allow two or more persons to simultaneously hear the acoustic signal. The converting means may be fitted with separate volume adjusters for the signals reproduced by said headsets.

There may be also provided an analog-to-digital converter (not shown) coupled to the connector 15 for converting the signal generated by the sensor 2 into a digital form. A digital signal analysis unit (not shown) may be connected to the output of said analog-to-digital converter.

In addition, the connector 15 may be connected to a computer (not shown) to be supplied with the signal generated by the acceleration sensor 2 for the purpose of analysing this signal, and/or comparing it to standard signals, and/or transmitting it via a communication link to another computer.

The connector 15 may be also connected to an external sound reproducer, and/or sound recorder, and/or graphic recorder represented e.g. by a loudspeaker, a tape recorder, an oscilloscope or an electrocardiograph (not shown).

The output terminals of the connector 15 intended for the connection of the above-listed external facilities, which may have high-voltage circuits, are coupled to the output of the filter 13 via an optoisolator 18.

It shall be evident that the electronic stethoscope may also comprise, apart from the above-mentioned devices, such conventional components of known electronic stethoscopes as an accumulator battery, a power supply unit, an on/off switch of push-button or sensor type, various light indicators etc.

In operation, the transducer 1 is brought into an acoustic contact with the surface of the object to be examined. The piezoelectric acceleration sensor 2 senses the accelerations transferred thereto from the object and arising under the action of acoustic oscillations generated within this object. After having received these accelerations, the sensor 2 converts the acoustic oscillations (vibrations) of the object surface into an electric signal.

In contrast with conventional microphones, the sensor 2 will not respond to acoustic oscillations coming to it through the air because it is devoid of any element, like a diaphragm, which could be set in motion under the effect of the variations in the air pressure. Also, because of a very low density of air, such oscillations will not cause vibrations of the sensor 2. Owing to the fact that the piezoelectric acceleration sensor 2 is essentially insensitive to the variations in the pressure of the ambient air, it becomes possible to achieve a high immunity of the electronic stethoscope to extraneous acoustic oscillations.

The piezoelectric elements 3 loaded with the inertial mass 4 sense accelerations in the directions shown in the drawings by arrows, i.e. perpendicular to the transducer face which is pressed against the object under examination, while the sensitivity of the sensor 2 to laterally directed accelerations is inferior to that observed in the main direction by about an order of magnitude. As a result, the sensor 2 shows a maximum sensitivity with respect to signals coming from sources located inside the object immediately under this sensor. Thanks to such a directed sensitivity of the sensor 2, the proposed stethoscope is capable of differentiating signals coming from different sound sources located inside the object under examination, such as various human internals.

The low density and the porous (matrix-type) texture of the ceramic material used for the piezoelectric elements 3 provide substantial increase in the sensitivity of the sensor in the audio frequency band, as well as reduction of its equivalent output resistance, thereby facilitating its matching with the amplifier input within a large audio frequency range and reducing the intrinsic noises introduced by the stethoscope. Such a reduction of the equivalent output resistance of the piezoelectric elements 3 is also achieved due to the parallel connection of two or several of them. As to the elastic protective cover 11, it ensures a good acoustic contact between the transducer 1 and the surface of the object under examination.

Due to the use of the sound-conducting rod 9, the sensor shown in Fig. 1 is easier to use for diagnosing various machines and devices, as well as for the search and rescue of victims in emergency situations, e.g. under the ruins of a building. The use, for such a sensor, of two or more piezoelectric elements mounted one over another provides increase in the sensor sensitivity caused by the increase in the total length of the sensing element.

Due to the in-phase combining of the output signals of several piezoelectric elements 3 circumferentially spaced on the inertial mass 4, the sensor shown in Fig. 2 possesses a sharper beam pattern with respect to the signals coming from the object being examined. Consequently, such a sensor is very suitable for use in individual diagnostic of various human internals. Since the center of gravity of the inertial mass 4 made in the form of a cup coincides with the center of gravity of the piezoelectric elements 3, the sensitivity of such a sensor can be brought up to its maximum possible level.

The electronic stethoscope in which the sensor 2 is located in a separate unit and the amplifying means are connected to said sensor 2 by the shielded cable 17, as shown in Fig. 2, is convenient to use for auscultation of a patient located in a separate chamber, such as a pressure chamber.

As can be seen in Figs. 1 and 2, the electric signal generated by the piezoelectric elements 3 is fed, via the electric lead 6 and the matching stage 14, to the input of the amplifier 12. Due to the possibility of controlling the gain of the amplifier 12, the desired level of the output signal may be achieved. As regards the passband of the filter 13, it is chosen on the basis of the particular characteristics of the acoustic oscillations being studied. Thus, for example, when diagnosing cardiac activity, this passband may be set to the range of about 30 to 300 Hz, whereas for auscultation of respiratory murmurs, it may range from 30 Hz to 5 kHz and higher. The provision, at the output of the connector 15, of the electroacoustic converting means coupled to the headset makes possible individual listening to the acoustic oscillations under study. The provision of two or more telephones connected to individual headsets, e.g. via a multichannel amplifier, allows two or more persons to simultaneously hear the acoustic signal to make possible taking collective decisions or training medical personnel. The same goals may be achieved by coupling to the connector 15 an external sound-reproducing apparatus, such as a power amplifier equipped with a loudspeaker.

In addition, there may be provided at the output of the connector 15 various external sound-recording and graphic recording devices, such as a tape recorder, an oscilloscope or an electrocardiograph, in order to acquire comprehensive information about the patient's pathology and its history, as well as to carry out training personnel.

The analog-to-digital converter (ADC) coupled to the connector 15 performs the conversion of the signal generated by the sensor into a digital form, whereas the digital signal analysis unit connected to the output of said analog-to-digital converter allows the signal parameters to be analyzed in accordance with a predetermined program, e.g. by comparing the obtained phonograms with standard ones.

In order to analyse the signals under study and to compare them with standard ones, the output of the connector 15 may be connected to a computer equipped with an ADC unit and appropriate software means. With such an arrangement, the signal generated by the acceleration sensor 2 can be transmitted via a communication link, using a modem, to another computer located e.g. in the room of the physician in charge or at the diagnostic center.

Due to the fact that the output terminals of the connector 15 intended for the connection of the above-listed external facilities having high-voltage circuits are coupled to the output of the filter 13 via the optoisolator 18, the stethoscope is safe to use, because in case of an insulation breakdown in the external facilities, the mains voltage will not appear at the sensor surface being in contact with the patient's body or the hands of the examiner.

It is to be understood that the invention is not limited to the above preferred embodiments of the electronic stethoscope design and that various other known structures of acceleration sensors, as well as other known means for processing, reproducing and analysing electric signals generated by the sensors may be used therein.

## Claims

1. An electronic stethoscope for examination of acoustically active objects, comprising an acoustical-electrical transducer (1) capable of being brought into an acoustic contact with the object surface, characterized in that said transducer (1) includes a piezoelectric acceleration sensor (2) adapted for sensing accelerations transferred to it from the object and arising under the action of acoustic oscillations generated in said object.

2. An electronic stethoscope according to Claim 1, characterized in that said sensor (2) includes at least one piezoelectric element (3) loaded with an inertial mass (4).

3. An electronic stethoscope according to Claim 2, characterized in that said at least one piezoelectric element (3) is made of porous ceramic material.

4. An electronic stethoscope according to Claim 3, characterized in that the proportion of pores volume is at least about 20 % of the overall ceramics volume in said piezoelectric element (3).

5. An electronic stethoscope according to any one of Claims 2 to 4, characterized in that said inertial mass (4) is a cup bearing on its bottom said at least one piezoelectric element (3).

6. An electronic stethoscope according to any one of the preceding Claims, characterized in that said piezoelectric acceleration sensor (2) comprises a plurality of piezoelectric elements (3) circumferentially spaced at regular intervals on said inertial mass (4) and electrically connected with each other.

7. An electronic stethoscope according to any one of the preceding Claims, characterized in that said piezoelectric acceleration sensor (2) comprises at least two piezoelectric elements (3) mounted on said inertial mass (4) one over the other and electrically connected with each other.

8. An electronic stethoscope according to Claim 6 or 7, characterized in that said piezoelectric elements (3) are connected in parallel.

9. An electronic stethoscope according to any one of the preceding Claims, characterized in that said transducer (1) is provided with a sound-conducting rod (9) one end of which is intended to be brought into contact with the object to be examined and the other end of which is in an acoustic contact with said piezoelectric acceleration sensor (2).

10. An electronic stethoscope according to any one of the preceding Claims, characterized in that the transducer surface intended to be brought into an acoustic contact with the surface of the object is equipped with an elastic protective cover (11).

11. An electronic stethoscope according to any one of the preceding Claims, characterized in that it comprises means (13) for frequency filtering the signal delivered by said sensor (2).

12. An electronic stethoscope according to Claim 11 characterized in that said frequency filtering means (13) have a switch-selectable passband.

13. An electronic stethoscope according to any one of the preceding Claims, characterized in that it comprises means (12) for amplifying the signal delivered by said sensor (2).

14. An electronic stethoscope according to Claim 13, characterized in that said amplifying means (12) have a controllable gain.

15. An electronic stethoscope according to Claim 13 or 14, characterized in that it comprises means for converting the signal amplified by said amplifying means (12) into an acoustic signal.

16. An electronic stethoscope according to Claim 15, characterized in that it comprises a headset for listening to the acoustic signal generated by said converting means.

17. An electronic stethoscope according to Claim 16, characterized in that said converting means comprise a telephone, said headset including olives acoustically coupled with said telephone via sound-conducting tubes.

18. An electronic stethoscope according to any one of Claims 11 to 17, characterized in that said converting means comprise at least one additional telephone connected to a separate headset so as to allow two or more persons to simultaneously hear the acoustic signal,

19. An electronic stethoscope according to any one of the preceding Claims, characterized in that it comprises an analog-to-digital converter for converting the signal generated by said sensor (2) into a digital form, and a digital signal analysis unit connected to the output of said analog-to-digital converter.

20. An electronic stethoscope according to any one of the preceding Claims, characterized in that it is adapted for supplying the signal generated by said acceleration sensor (2) to a computer for the purpose of analysing this signal, and/or comparing it to standard signals, and/or transmitting it via a communication link to another computer.

21. An electronic stethoscope according to any one of the preceding Claims, characterized in that it is adapted for feeding the signal generated by said acceleration sensor (2) to an external sound reproducer, and/or sound recorder, and/or graphic recorder.

22. An electronic stethoscope according to any one of the preceding Claims, characterized in that it is provided with output terminals to which the signal generated by said sensor (2) is supplied via an optoisolator (18).

23. An electronic stethoscope according to any one of Claims 13 to 22, characterized in that said piezoelectric acceleration sensor (2) is located inside a separate unit, said amplifying means (12) being connected to said sensor (2) by a shielded cable (17).
